# EUROPÄISCHE PATENTSCHRIFT

(11) **EP 0 528 214 B1**
(45) Veröffentlichungstag und Bekanntmachung des Hinweises auf die Patenterteilung: **22.01.1997**
(21) Anmeldenummer: 92112840.1
(22) Anmeldetag: 28.07.1992
(51) Int. Cl.: C07F 5/02, C07D 487/22, C07C 13/62

(54) **Verfahren zur Herstellung von Heterofullerenen und Fullerenen**
Process for the preparation of heterofullerenes and fullerenes
Procédé de préparation d'hétérofullerènes et fullerènes

(30) Priorität: 17.08.1991 DE 4127268; 27.08.1991 DE 4128357
(43) Veröffentlichungstag der Anmeldung: 24.02.1993
(73) Patentinhaber: Keesmann, Till, D-69115 Heidelberg (DE)
(72) Erfinder: Oeste, Franz Dietrich, W-6309 Münzenberg (DE)
(74) Vertreter: Clemens, Gerhard, Dr.-Ing.

(56) Entgegenhaltungen:
- DE-A- 4 114 536
- NATURE Bd. 352, Nr. 6331, 1991, Seiten 139 - 141 J.B. HOWARD ET AL

## Beschreibung

Die vorliegende Erfindung betrifft ein Verfahren zur Herstellung von aus geschlossenem Hexagon- und Pentagonnetzwerk bestehenden Hohlmolekülen.

In der DE-A-4 114 536, die nach Artikel 54(2) EPÜ nicht zum Stand der Technik gehört, wird über die Herstellung von Kohlenstoff, Stickstoff und Bor enthaltenden Heterofullerenen berichtet. Danach werden diese Heterofullerene, die hier als CBN-Heterofullerene bezeichnet werden, durch die Reaktion von Edukten in dichten fluiden Phasen wie Flüssigkeiten oder komprimierten Gasen mit flüssigkeitsähnlichen Dichten hergestellt bei Temperaturen, bei denen keine Pyrolyse eintritt.

Nach einer zusammenfassenden Darstellung, die in der Zeitschrift New Scientist vom 6.7.1991, Seite 26 und Seiten 34 bis 38 erschienen ist, werden Fullerene aus physikalisch erzeugtem Kohlenstoffdampf in einer Hochtemperatursynthese hergestellt. Nach einer Mitteilung, die in der Zeitschrift Nature, Nr. 352 vom 11.7.1991, Seiten 139 bis 141, erschienen ist, können Fullerene auch aus den Feststoffen gewonnen werden, die bei der unvollständigen Verbrennung von einfachen Kohlenwasserstoffen in einer Flamme entstehen.

Sowohl mit der Flammensynthese als auch mit den verschiedenen Hochtemperatursynthesen waren bislang nur Fullerene darstellbar in wägbaren Ausbeuten mit Kohlenstoffzahlen unter 100. Die gezielte Synthese von Fullerenen mit Kohlenstoffzahlen über 100 oder gar über 1000 nach diesen Verfahren wird nicht beschrieben. Ebenfalls wird die Synthese von CBN-Heterofullerenen oder gar die gezielte Synthese von CBN-Heterofullerenen mit einer bestimmten Atomzahl oder mit einer bestimmten Molekülsymmetrie mittels Plammen- oder Hochtemperatursynthese nicht berichtet.

Weil die o.g. Synthese der Heterofullerene wegen der Vielzahl von Syntheseschritten aufwendiger sein kann als die Flammen- oder Hochtemperatursynthese, kann es wirtschaftlich vorteilhafter sein, für die Synthese der CBN-Heterofullerene ebenfalls die Hochtemperatursyntheseverfahren oder die Flammensynthese anzuwenden. Es besteht daher die Aufgabe, ein Verfahren zur Herstellung von CBN-Heterofullerenen, zur gezielten Herstellung von CBN-Heterofullerenen mit bestimmter Atomzahl und bestimmter Symmetrie und zur gezielten Herstellung von Fullerenen mit bestimmter Kohlenstoffzahl und bestimmter Symmetrie mit der Flammen- oder Hoch-temperatursynthese zu finden.

Diese Aufgabe wurde durch die vorliegende Erfindung gelöst. Danach wurde überraschend gefunden, daß dann, wenn die Elemente Kohlenstoff, Bor und Stickstoff als solche und/oder in Form bestimmter Verbindungen der Flammen- oder Hochtemperatursynthese ausgesetzt werden, je nach den gewählten Verhältnissen die Herstellung von CBN-Heterofullerenen, die gezielte Herstellung von CBN-Heterofullerenen mit bestimmter Atomzahl und bestimmter Symmetrie oder die gezielte Herstellung von Fullerenen mit einer bestimmten Kohlenstoffzahl und bestimmter Symmetrie gelingt.

Das erfindungsgemäße Erfinden zur Herstellung von aus geschlossenem Hexagon- und Pentagonnetzwerk bestehenden Hohlmolekülen, zeichnet sich dadurch aus, daß durch Pyrolyse aus Edukten Hohlmoleküle hergestellt werden,
a) die Kohlenstoff und Bor und/oder Stickstoff als Bestandteile des Hexagon- und Pentagonnetzwerkes enthalten, wobei Edukte verwendet werden, die diese Elemente enthalten oder
b) die Kohlenstoff und Bor und/oder Stickstoff als Bestandteile des Hexagon- und Pentagonnetzwerkes enthalten und bei denen Molekülsymmetrie- und Molekülabmessungsparameter mit denen von mit Öffnungen im Hexagon- und Pentagonnetzwerk versehenen Hohlmolekülen von Edukt bestandteilen übereinstimmen, wobei Edukte verwendet werden, die Kohlenstoff und Bor und/oder Stickstoff und ein oder mehrere Fullerenfragmentderivate enthalten oder Edukte verwendet werden, die Kohlenstoff und Bor und/oder Stickstoff und ein oder mehrere, Kohlenstoff und Bor und/oder Stickstoff enthaltende Heterofullerenfragmentderivate enthalten oder
c) die ausschließlich Kohlenstoff als Bestandteil des Hexagon- und Pentagonnetzwerkes enthalten und bei denen Molekülsymmetrie- und Molekülabmessungsparameter mit denen von mit Öffnungen im Hexagon- und Pentagonnetzwerk versehenen Hohlmolekülen von Eduktbestandteilen übereinstimmen, wobei Edukte verwendet werden, die Kohlenstoff und ein oder mehrere Fullerenfragmentderivate enthalten und
d) die Trennung des die Hohlmoleküle enthaltenden festen Reaktionsgemisches mittels üblicher Trenntechniken, insbesondere Extraktion mit organischen Lösungsmitteln und/oder durch Sorption, erfolgt.

Erfindungsgemäß sind alle diejenigen thermischen Verfahren zur Erzeugung der CBN-Heterofullerene oder der CBN-Heterofullerene und Fullerene, bei denen Molekülsymmetrie- und Molekülabmessungsparameter mit denen von Eduktbestandteilen übereinstimmen, geeignet, die die Kohlenstoff, Stickstoff und/oder Bor enthaltenden Stoffe in reaktive gasförmige und/oder aerosolförmige Verbindungen überführen.

Hierzu zählen die genannten Hochtemperatur-Syntheseverfahren, die bei Temperaturen ablaufen, bei denen elementares Bor oder Kohlenstoff bereits merkliche Dampfdrücke aufweisen, aber auch Pyrolyseprozesse, wie sie z.B. in Flammen bei der mehr oder weniger unvollständigen Verbrennung oxidierbarer Kohlenstoff-, Bor- oder Stickstoffverbindungen mit Oxidantien wie Sauerstoff oder Stickoxiden auftreten.

Erfindungsgemäß werden die Heterofullerene und Fullerene vorzugsweise durch Extraktion mit organischen Lösungsmitteln aus den festen Reaktionsprodukten gewonnen. Ggf. ist die Extraktion mit überkritischen Lösungsmitteln angezeigt, wenn es sich um hochmolekulare Produkte handelt. Die Auftrennung der teilsweise komplexen isomerenhaltigen Gemische kann nach den üblichen Trenntechniken wie z.B. Chromatographie geschehen. Dieses Trennverfahren hat sich bereits zur Trennung der Fullerene C₆₀ und C₇₀ bewährt. Auf die Möglichkeit zur Gewinnung der CBN-Heterofullerene und Fullerene aus dem die Produkte enthaltenden Gasstrom durch Sorption wird ausdrücklich hingewiesen.

In der Tabelle 1 sind beispielhaft Edukte genannt, aus denen mit dem erfindungsgemäßen Verfahren die CBN-Heterofullerene hergestellt werden können.

In der Tabelle 2 sind beispielhaft Edukte genannt, aus denen mit dem erfindungsgemäßen Verfahren Fullerene mit bestimmten eduktanalogen Symmetrie- und Abmessungsparametern hergestellt werden können.

Die in den Tabellen genannten CBN-Heterofullerenfragmentderivate und Fullerenfragmentderivate werden hinsichtlich Herstellung und Aufbau ausführlich in der DE-A-4 114 536 beschrieben. Hier sei nur darauf hingewiesen, daß diese Fragmentderivate hinsichtlich Radius und Symmetrieeigenschaften den CBN-Heterofullerenen bzw. Fullerenen gleichen, daß sie aber zum Unterschied zu diesen Öffnungen in der Molekülschale enthalten, deren Ränder mit verschiedenen Funktionen besetzt sind, die zB. Wasserstoff oder Stickstoff enthalten.

**Tabelle 1**

| lfd.Nr. | Edukt | Edukt geeignet als Quelle für | | | bevorgzugte Eignung | |
|---|---|---|---|---|---|---|
| | | Kohlenstoff | Bor | Stickstoff | Flammen- | Hochtemp.- |
| | | in CBN-Heterofullerenen | | | synthese | |
| 1 | Bor | | x | | | x |
| 2 | Kohlenstoff | x | | | | x |
| 3 | Stickstoff | | | x | | x |
| 4 | Kohlenswasserstoffe | x | | | x | x |
| 5 | Borazin | | x | x | x | x |
| 6 | Hexamethylborazin | x | x | x | x | x |
| 7 | Bornitrid | | x | x | | x |
| 8 | Borcadib | x | x | | | x |
| 9 | Pentoboran | | x | | x | x |
| 10 | 1-Pyrrolylboran | x | x | x | x | x |
| 11 | Stickoxide | | | x | x | |
| 12 | Ammoniakgas | | | x | x | x |
| 13 | Harnstoff | x | | x | x | x |
| 14 | CBN-Heterofullerenfragmentderivate | x | x | x | x | x |
| 15 | Fullerenfragmentderivate | x | | x | x | x |

**Tabelle 2**

| lfd. Nr. | Edukte mit Eignung als Kohlenstoffquelle für Fullerene | bevorzugte Eignung | |
|---|---|---|---|
| | | Flammen- | Hochtemp.- |
| | | synthese | |
| 1 | Kohlenstoff | | x |
| 2 | BTX-Aromaten | x | x |
| 3 | Acetylen | x | x |
| 4 | aromatische Rußöle | x | |
| 5 | Fullerenfragmentderivate | x | x |

Zu den in Tabelle 2 aufgezählten Edukten ist anzumerken, daß nach dem erfindungsgemäßen Verfahren zur Herstellung von Fullerenen mit bestimmten eduktidentischen Symmetrie- und Abmessungsparametern die Kohlenstoffquellen immer zusammen mit einem Fullerenfragmentderivat eingesetzt werden müssen.

Zu den in Tabelle 1 aufgezählten Edukten ist anzumerken, daß nach dem erfindungsgemäßen Verfahren zur Herstellung von CBN-Heterofullerenen mit bestimmten eduktidentischen Symmetrie- und Abmessungsparametern die Kohlenstoff- und/oder Bor- und/oder Stickstoff-Quellen immer zusammen mit einem Fullerenfragmentderivat oder mit einem CBN-Heterofullerenfragmentderivat eingesetzt werden müssen. Die nicht eduktgesteuerte Herstellung von CBN-Heterofullerenen gelingt dagegen ohne die Verwendung der unter 14. oder 15. genannten Edukte mit anderen der beispielhaft genannten C-, N- und B-Quellen.

### Beispiel 1

In Anlehnung an die Methode zur Herstellung von borhaltigen Subphthalocyaninen, die in der Zeitschrift Monatshefte für Chemie, Nr. 103, Jahrgang 1972, Seiten 150 bis 155, mitgeteilt wird, werden in einem Rührautoklaven 400 g Triphenylboran, die von der Aldrich-Chemie, D-7924 Steinheim bezogen werden können, und 800 ml Benzol auf eine Temperatur von 300 °C und einen Druck von 350 bar gebracht. Zu dieser Lösung werden langsam 50 g des Tetranitrils 1), das entsprechend der Vorschrift, die in der DE-A- 4 114 536 veröffentlicht ist, zuvor hergestellt 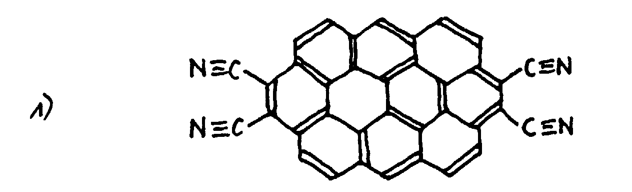 wird, in 1000 ml Benzol gelöst zugegeben. Nach 1 h Reaktionszeit wird 15 min lang absitzen lassen und dann die überstehende fluide Phase abgezogen, entspannt und abgekühlt. Dabei scheidet sich aus der abgezogenen Phase ein brauner feinteiliger Niederschlag aus, bei dem es sich um das CBN-Heterofullerenfragmentderivat C₂₄₀H₈₀N₂₄B₄ mit tetraedersymmetrischer Molekülsymmetrie handelt, das durch die Formel 2) ausschnitthaft beschrieben wird. Danach entspricht das CBN-Heterofullerenfragmentderivat hinsichtlich Symmetrie und Tetraeder-Abmessung dem tetraedersymmetrischen Fulleren C₂₉₂ mit dem Hexagon-Pentagon-Arrangement je Tetraederecke, das in der Formel 3) dargestellt ist.

100 g des CBN-Heterofullerenfragmentderivats 2) werden mit 1000 ml Benzol und 200 ml Triphenylboran in einem Rührautoklaven auf 300 °C und 320 bar gebracht und ca. 30 min belassen, bis sich eine homogene Lösung gebildet hat. Diese Lösung wird 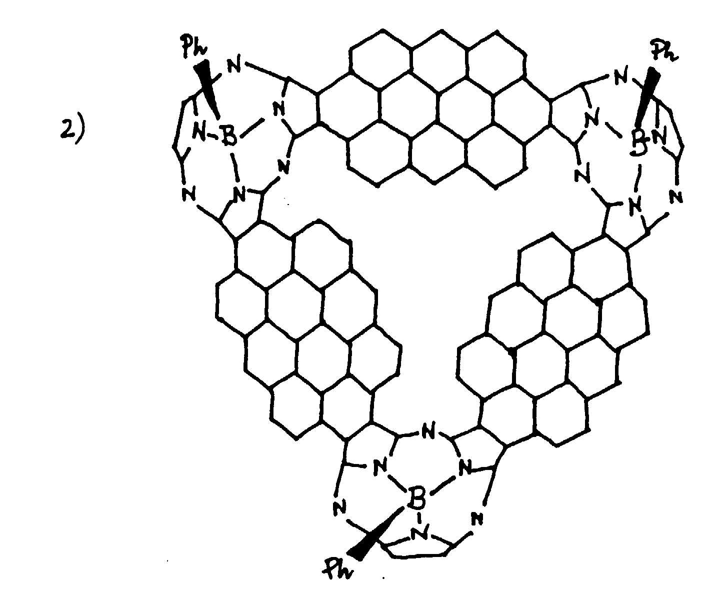 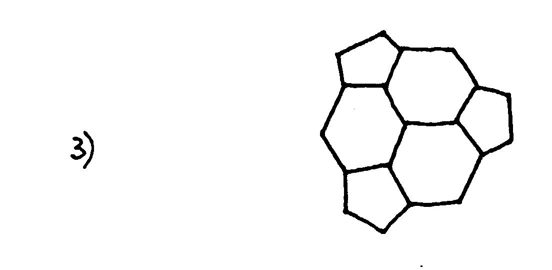 mit einer Durchflußrate von ca. 1 l/h mittels einer Düse in einen Brennraum hinein entspannt und dort entzündet. Dazu wird Luft als Oxidationsmittel unterstöchiometrisch so zugegeben, daß eine stark rußende stabile Flamme entsteht. Die Brennkammer ist dabei auf einen Unterdruck von 100 bis 200 Torr evakuiert. Die abgesaugten festen Verbrennungsprodukte werden in einem Quarzfaservlies-Filter aufgefangen. Danach werden die festen Verbrennungsprodukte so quantitativ wie möglich eingesammelt und einschließlich des Quarzvliesfilters zusammen mit 500 ml Benzol in einem Rührautoklaven 50 min lang bei 300 °C und 320 bar behandelt. Danach wird 15 min lang absitzen lassen und dann das überstehende Fluid abgezogen, abgekühlt und entspannt. Dabei fällt aus der abgezogenen Phase ein brauner Niederschlag aus, der zu etwa 30 % aus dem tetraedersymmetrischen CBN-Heterofulleren C₂₆₀N₂₄B₈ besteht, das die gleiche Atomzahl enthält wie das entsprechende tetraedersymmetrische Fulleren C₂₉₂. Das CBN-Heterofulleren C₂₆₀N₂₄B₈ wird ausschnitthaft in der Formel 4) gezeigt.

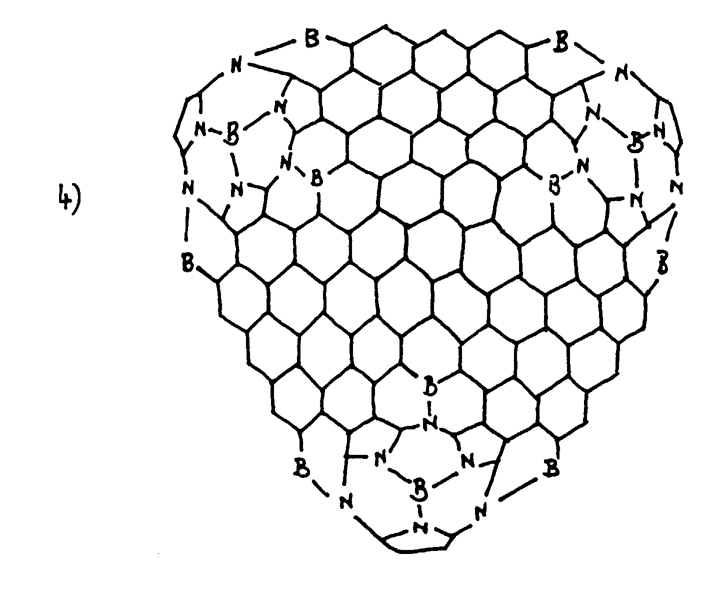

Auf die Darstellung von Doppelbindungen wird bei den Formeldarstellungen in der Regel verzichtet. Durch die Formeldarstellung soll nur die Koordinationssituation zwischen den beteiligten Elementen verdeutlicht werden. Wasserstoffunktionen werden nicht dargestellt. Ebenso wird bei den folgenden Beispielen verfahren.

### Beispiel 2

In Anlehnung an eine Methode zur Darstellung von borhaltigen Subphthalocyaninen, die in der Zeitschrift Monatshefte für Chemie, Nr. 103, Jahrgang 1972, Seiten 150 bis 155, mitgeteilt wird, werden in einem Rührautoklaven 400 g Triphenylboran und 1000 ml Benzol auf eine Temperatur von 260 °C und einen Druck von 300 bar gebracht. Zu dieser Lösung wird langsam eine Lösung von 20 g Tetracyanethylen in 500 ml Benzol zugegeben. Danach beläßt man noch 1 h unter Reaktionsbedingungen, läßt dann 15 min absitzen und zieht danach die überstehende fluide Phase ab. Aus der abgezogenen entspannten und abgekühlten Phase fällt ein dunkelbraunes Pulver aus, bei dem es sich um das tetraedersymmetrische CBN-Heterofullerenfragmentderivat mit der Summenformel C₆₀H₂₀N₂₄B₄ handelt und das in den Formeln 5) und 6) in verschiedenen Molekülansichten ausschnitthaft gezeigt wird. Damit entspricht das

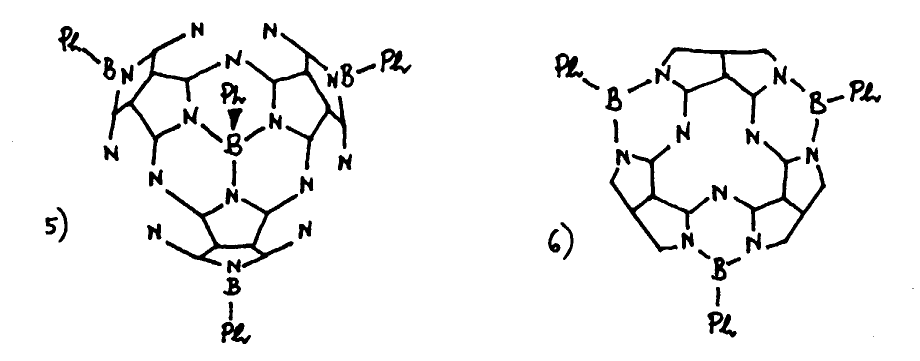

CBN-Heterofullerenfragmentderivat hinsichtlich Symmetrie und Tetraederabmessung dem tetraedersymmetrischen Fulleren C₆₈, das das gleiche Hexagon-Pentagon-Arrangement je Tetraederecke aufweist. Das Arrangement wird in der Formel 3) gezeigt.

100 g des CBN-Heterofullerenfragmentderivates wird zusammen mit 500 ml Benzol und 500 ml Borazin in einem Rührautoklaven auf 300 °C erwärmt und unter 320 bar Druck gebracht und 30 min dabei belassen bis sich eine homogene Lösung gebildet hat. Diese Lösung wird mit einer Durchflußrate von ca. 1 l/h entsprechend Beispiel 1 verbrannt und die festen Verbrennungsprodukte entsprechend Beispiel 1 aufgearbeitet. Bei dem erhaltenen braunen Niederschlag handelt es sich zu ca. 20 % um das CBN-Heterofulleren C₃₆N₂₄B₈, das die gleiche Atomzahl enthält wie das Fulleren C₆₈. Sowohl das CBN-Heterofulleren als auch das Fulleren haben die gleiche tetraedersymmetrische Konfiguration und enthalten das gleiche Hexagon-Pentagon-Arrangement je Tetraederecke entsprechend Formel 3). Zwei verschiedene Molekülansichten des CBN-Heterofullerens werden in den Formelausschnitten 7) und 8) gezeigt.

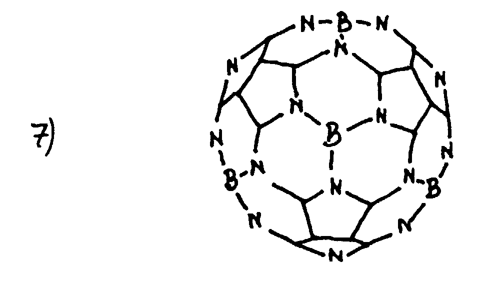

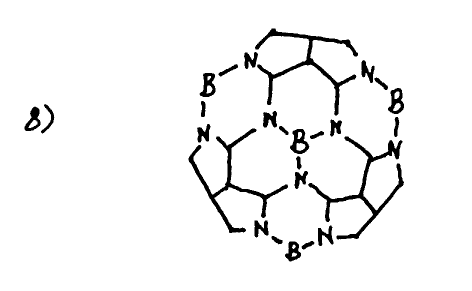

### Beispiel 3

100 g des oktaedersymmetrischen Fullerenfragmentderivates C₁₆₈H₁₀₈N₂₄, dessen Molekülaufbau aus den beiden Formelbildern 9) und 10) hervorgeht, die jeweils eine andere Ansicht des Moleküls zeigen, das nach Molekülsymmetrie dem oktaedersymmetrischen Fulleren C₂₇₆ entspricht und das entsprechend der in der DE-A-4 114 536 veröffentlichten Vorschrift hergestellt wird, wird mit 1000 g Benzol in einem Rührautoklaven auf 300 ^{o}C erwärmt und auf 320 bar verdichtet. Nach der vollständigen Lösung wird der Autoklaveninhalt entsprechend Beispiel 1 in eine Brennkammer entspannt und entsprechend verbrannt. Die Aufarbeitung der festen Verbrennungsprodukte geschieht ebenfalls entsprechend Beispiel 1. Als Produkt fällt ein braunroter Niederschlag an, der etwa 15 % des oktaedersymmetrischen Fullerens C₂₇₆ enthält. Das Fulleren, dessen Molekülradius etwa dem des eingesetzten Fullerenfragmentderivats entspricht, wird ausschnitthaft in der Formel 11) gezeigt und zwar mit der Blickrichtung auf eine der Oktaederecken des Fullerens, die offenbar aus den sechs Porphinfunktionen des Fullerenfragmentderivates entstanden sind entsprechend Schema 12).

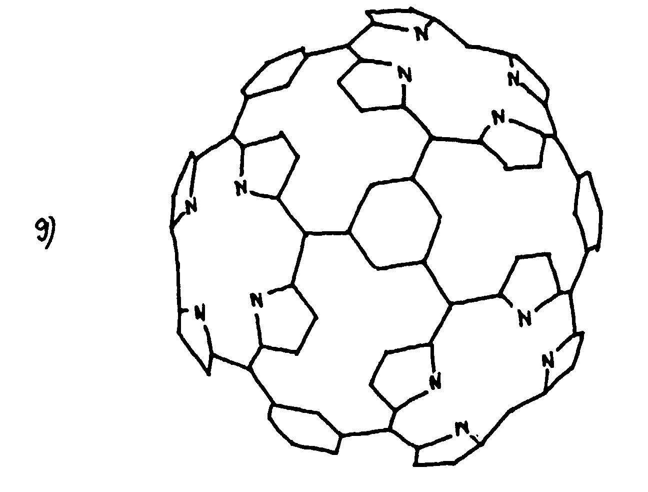

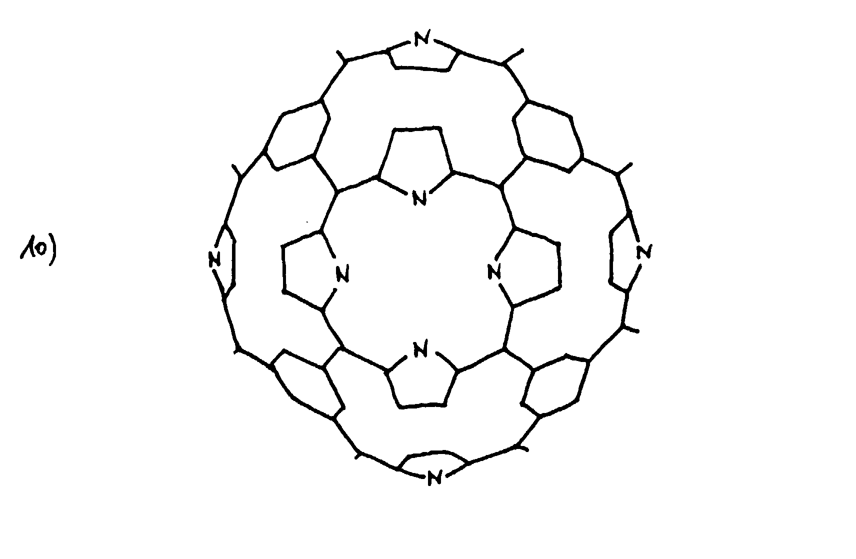

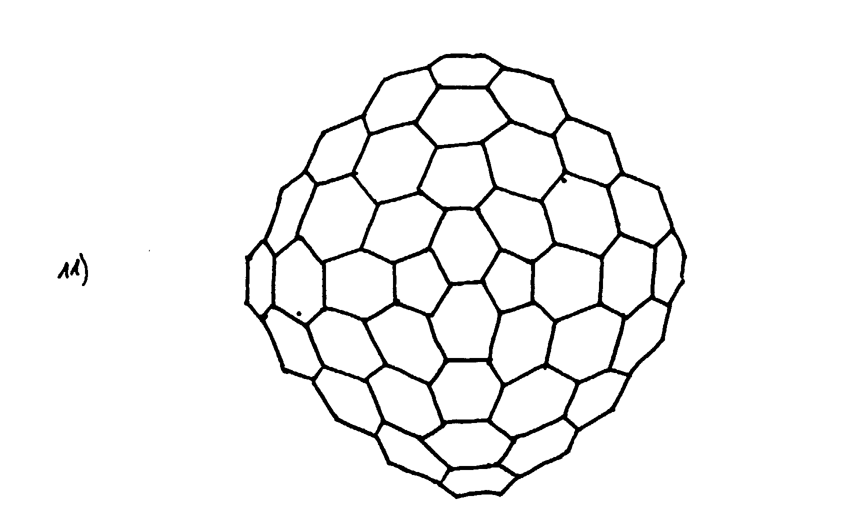

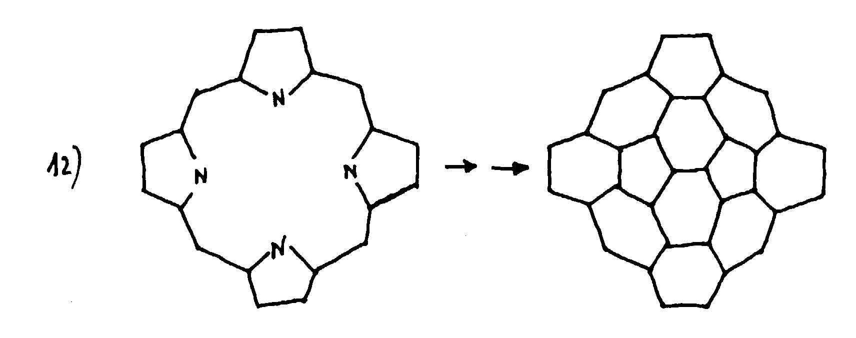

### Beispiel 4

Beispiel 3 wird wiederholt jedoch mit dem Unterschied, daß anstelle von Benzol zur Verbrennung ein Gemisch von 700 g Hexamethylborazin, 300 g Benzol und 50 g Ammoniakgas eingesetzt wird. Der Anteil des eingesetzten Fullerenfragment derivates C₁₆₈H₁₀₈N₂₄ bleibt dagegen unverändert. Als Produkt fällt ein braunrötliches Pulver an, das mindestens zu einem Viertel aus CBN-Heterofullerenen mit jeweils 276 Atomen je Molekül und oktaedersymmetrischer Molekülsymmetrie besteht, wobei die CBN-Heterofullerene offensichtlich unterschiedliche C-, B- und N-Gehalte aufweisen. Entsprechend dem durchschnittlichen C-, N- und B-Gehalt wird einem Großteil des Produktes die Formel C₂₂₈N₂₄B₂₄ zugesprochen entsprechend dem in der Formel 13) ausschnitthaft mit Blickrichtung auf eine Oktaederecke wiedergegebenen Molekül.

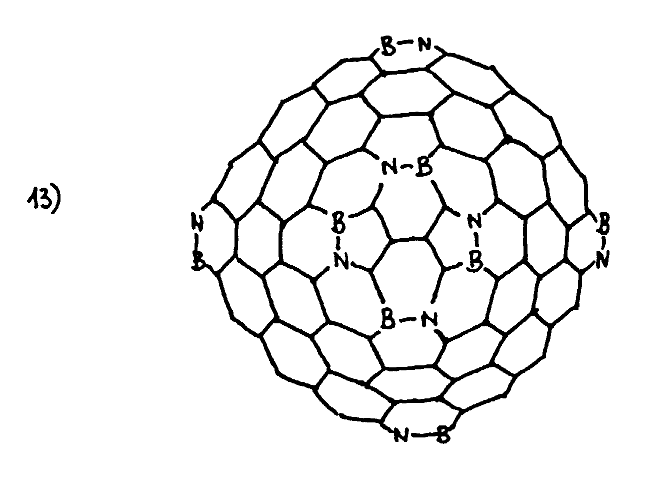

### Beispiel 5

Beispiel 4 wird wiederholt mit dem Unterschied, daß als Oxidationsmittel anstelle von Luft NO in unterstöchiometrischem Verhältnis zu dem Brennstoff eingesetzt wird und daß die Brennstoffzusammensetzung dahingehend verändert wird, daß neben dem Anteil des Fullerenfragmentderivates C₁₆₈H₁₀₈N₂₄ 500 g Hexamethylborazin und 500 g Benzol verwendet werden. Es wird dabei ein Produkt erhalten, das im Wesentlichen dem von Beispiel 4 entspricht.

### Beispiel 6

Beispiel 3 wird wiederholt mit dem Unterschied, daß das Eduktgemisch nicht der Verbrennung sondern einer Hochtemperaturpyrolyse zugeführt wird. Dazu wird das unter Druck stehende Eduktgemisch über eine Düse in einen Argonstrom feinteilig vernebelt, so daß dieser eine Beladung erhält von 8 g/m³ Benzol und 0,6 g/m³ des Fullerenderivates C₁₆₈H₁₀₈N₂₄. Der Argonstrom wird danach durch ein Graphitrohr geleitet, das von zwei wassergekühlten Kupferklemmen gehalten wird und das als elektrischer Widerstand geschaltet ist und mittels Wechselstrom auf ca. 2500 °C erwärmt ist. Die Aufenthaltszeit des Argonstroms im heißen Rohr beträgt ca. 1 s. Die festen Reaktionsprodukte werden wie in Beispiel 3 beschrieben gesammelt und aufgearbeitet. Auch in diesem Fall fällt ein braunroter feinteiliger Niederschlag nach der Extraktaufarbeitung an, der etwa 10 % des oktaedersymmetrischen Fullerens C₂₇₆ enthält, das auch in Beispiel 3 gewonnen wurde. Es ist nachzutragen, daß die Hochtemperaturpyrolyse im Vakuum bei 100 bis 200 Torr vorgenommen wird.

### Beispiel 7

Unter den Bedingungen, die in Beispiel 6 beschrieben werden, wird ein Argongasstrom durch Hindurchleiten von Teilströmen bei Normaldruck durch Gaswaschflaschen, die die betreffenden Flüssigkeiten enthalten, erhalten, der bei 20 ^{o}C beladen ist mit 8 g/m³ Benzol, 2 g/m³ Borazin und 2 g/m³ Pyridin. Vor dem Eintritt in das heiße Graphitrohr wird das beladene Argongas auf 100 bis 200 Torr entspannt. Die Aufarbeitung der festen Reaktionsprodukte geschieht durch einfache Soxhlet-Extraktion mit Toluol und Eindampfen des Extraktes. Der feste bräunliche Rückstand enthält neben Spuren von Fullerenen C₆₀ und C₇₀ einen überwiegenden Anteil von CBN-Heterofullerenen mit Radien und Atomzahlen die den Fullerenen C_{<100} entsprechen. Die entstandenen CBN-Heterofullerene enthalten auch das CBN-Heterofulleren C₃₆N₂₄B₈, das in dem Beispiel 2 gezielt mit weit höherer Ausbeute hergestellt wurde, und das die beiden Formeln 7) und 8) ausschnitthaft zeigen.

### Beispiel 8

800 g Benzol, 100 g Triphenylboran und 60 g des CBN-Heterofullerenderivates C₂₄₀H₈₀N₂₄B₄, das entsprechend Beispiel 1 hergestellt wurde, werden in einem Rührautoklaven bei 280 °C und 320 bar gelöst und über eine Düse als feines Aerosol in einen Argonstrom hineingesprüht, so daß dieser eine Beladung von 8 g/m³ Benzol, 1 g/m³ Triphenylboran und 0,6 g/m³ C₂₄₀H₈₀N₂₄B₄ erhält. Vor Eintritt in das erwärmte Graphitrohr entsprechend Beispiel 6, wird der beladene Argonstrom auf einen Druck von 100 bis 200 Torr entspannt. Das Abscheiden und Auffangen der festen Kondensationsprodukte, sowie deren Aufarbeitung erfolgt wieder entsprechend Beispiel 6. Das so gewonnene Produkt enthält als überwiegenden Heterofullerenanteil das in Beispiel 1 beschriebene CBN-Heterofulleren C₂₆₀N₂₄B₈.

### Beispiel 9

Beispiel 8 wird dahingehend verändert wiederholt, daß das Flüssigkeitsgemisch, mit dem der Argonstrom beladen wird, verändert wird. Unter sonst gleichen Zubereitungsbedingungen erhält die Argonbeladung folgende Zusammensetzung: 1 g/m³ Triphenylboran, 8 g/m³ Benzol, 0,4 g/m³ des Fullerenfragmentderivates C₁₀₈₀H₃₀₀N₁₂₀O₂₄U₁₂, dessen Radius und molekulare Symmetrie dem ikosaedrischen Fulleren C₁₆₂₀ entspricht. Das Fullerenfragmentderivat ist in der Formel 14) ausschnitthaft dargestellt. Nach der Elementarzusammensetzung des aus dem Extrakt isolierten Heterofullerenanteils besteht dieser zum überwiegenden Anteil aus dem CBN-Heterofulleren, das der Zusammensetzung C₁₃₈₀N₁₂₀B₁₂₀ nahekommt. Ein CBN-Heterofulleren, das dieser Zusammensetzung entspricht und ikosaedersymmetrischen Molekülaufbau hat, wie das Fulleren C₁₆₂₀, zeigt der Formelausschnitt 15). Der Formelausschnitt 15) zeigt das CBN-Heterofulleren mit Blickrichtung auf eine der 12 Ikosaederecken, die entsprechend Schema 16) aus den 12 Superphthalocyaninfunktionen des Fullerenfragmentderivates des Eduktes entstanden sind.

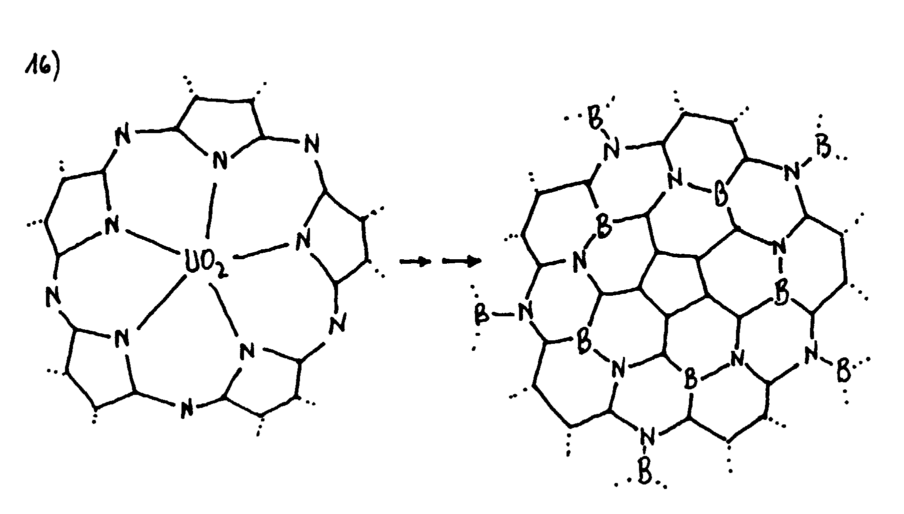

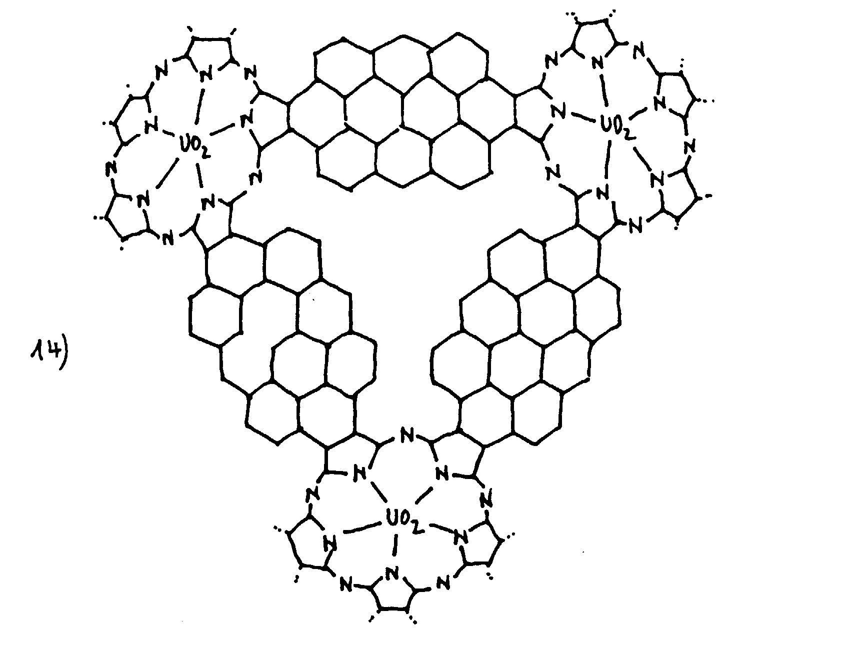

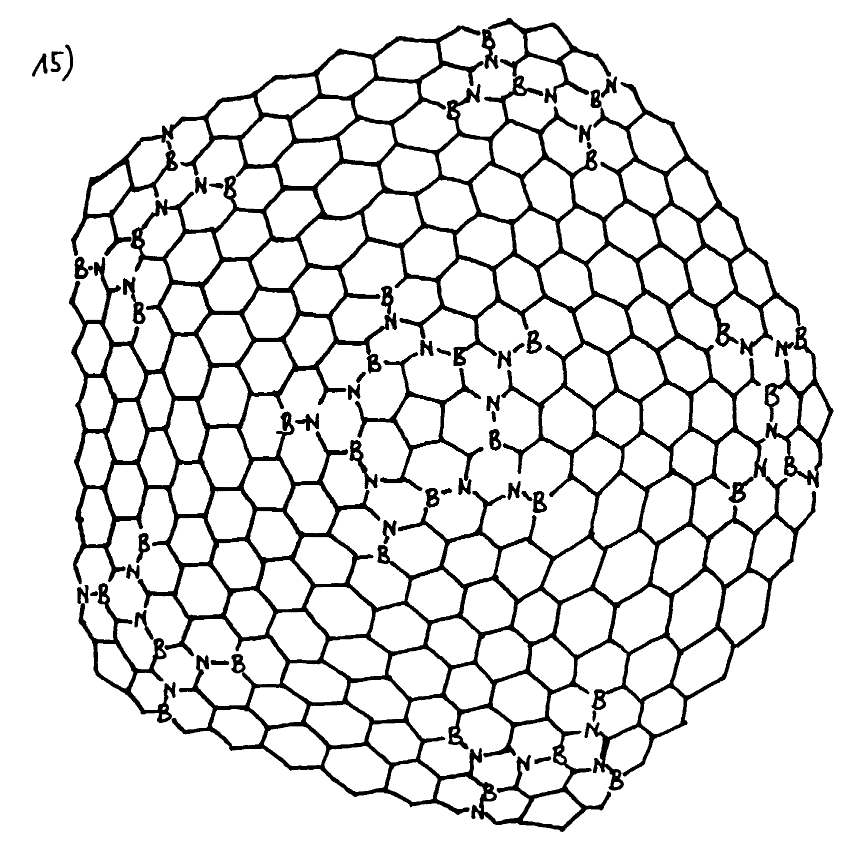

### Beispiel 10

In Anlehnung an eine Methode zur Herstellung von Subphthalocyanin-Derivaten, die in der Zeitschrift Monatshefte für Chemie, Nr. 103, Jahrgang 1972, Seiten 150 bis 155, mitgeteilt wird, entsprechend den Synthesebedingungen, die in der DE-A-4 114 536 mitgeteilt werden, wird aus 2,3,6,7,10,11-Hexacyanotriphenylen und Bortrichlorid das CBN-Heterofullerenfragmentderivat C₉₆H₂₄B₄Cl₄N₂₄ hergestellt, das nach Symmetrie und Käfigradius dem tetraedersymmetrischen Fulleren C₁₆₀ entspricht. Es ist in der Formel 17) ausschnitthaft dargestellt. Das B-Tetrachlorderivat des CBN-Heterofullerenfragmentderivats 17) wird mit Natriumborhydrid zu dem B-Tetrahydroderivat C₉₆H₂₈B₄N₂₄ 18) umgesetzt. 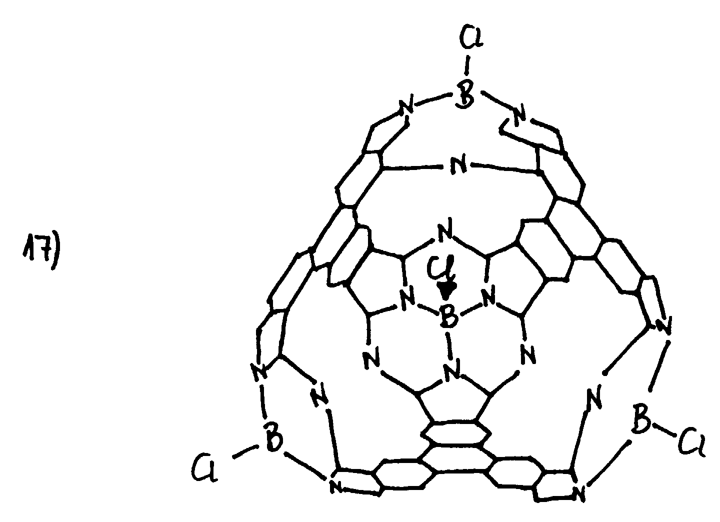 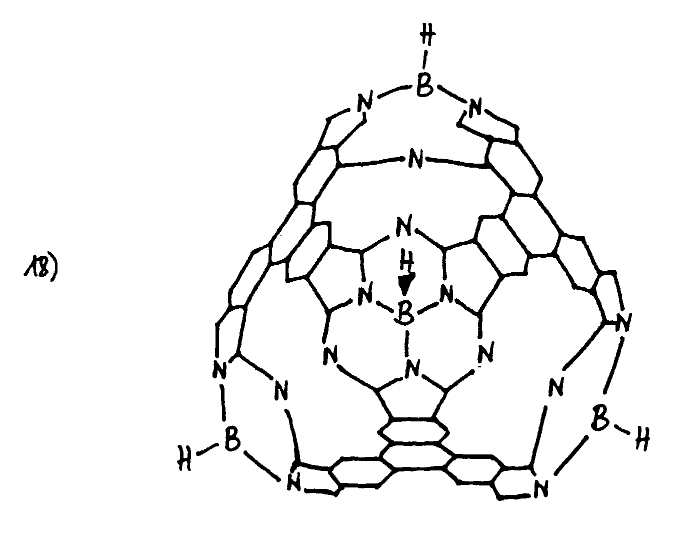 Anschließend wird die in Beispiel 8 beschriebene Prozedur der Hochtemperaturpyrolyse im Argonstrom mit veränderter Argonbeladung wiederholt: 6 g/m³ Borsäuremethylester, 8 g/m³ Toluol, 0,3 g/m³ des CBN-Heterofullerenfragmentderivates C₉₆H₂₈B₄N₂₄. Der entsprechend Beispiel 8 aufgearbeitete Extrakt der festen Hochtemperaturpyrolyseprodukte, eine hellbraune feinteilige Masse, enthält etwa einen gleich hohen Anteil des tetraedersymmetrischen CBN-Heterofullerens C₁₂₈B₈N₂₄, dessen Molekül ausschnitthaft in der Formel 19) gezeigt wird, und das nach Symmetrie und Atomzahl dem tetraedersymmetrischen Fulleren C₁₆₀ entspricht, und der Fullerene C₆₀ und C₇₀.

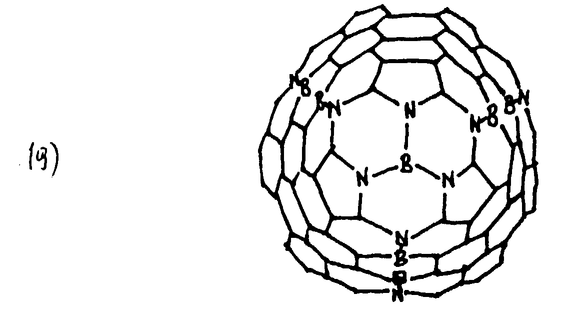

### Beispiel 11

Entsprechend der DE-A-4 114 536 wird aus Bortrichlorid und dem Hexaazatriphenylenhexacarbonitril 20) das CBN-Heterofullerenfragmentderivat C₇₂B₄Cl₄N₄₈ hergestellt. Dieser Molekülkäfig, der hinsichtlich Symmetrie und Käfigradius dem tetraedersymmetrischen Fulleren C₁₆₀ entspricht, ist ausschnitthaft in der Formel 21) dargestellt. 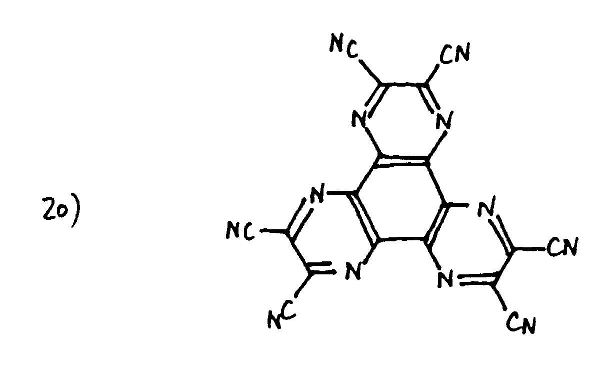 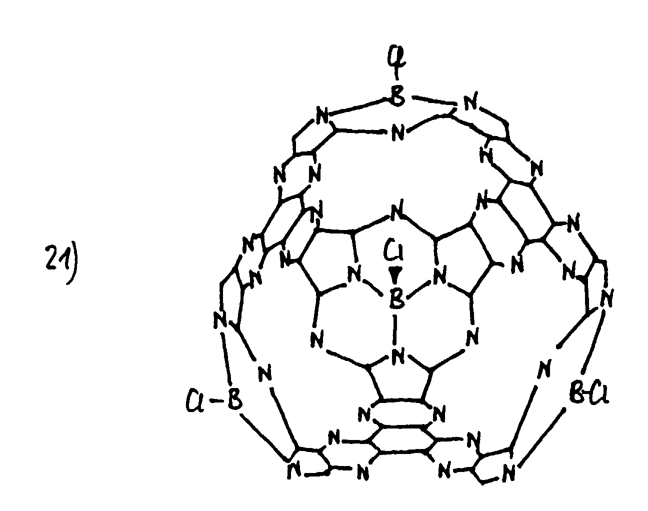 Das B-Tetrachloroderivat 21) wird mittels Natriumborhydrid in das B-Tetrahydroderivat C₇₂H₄B₄N₄₈ reduziert. Anschließend wird die in Beispiel 8 beschriebene Prozedur der Hochtemperaturpyrolyse mit folgender Argonbeladung wiederholt: 8 g/m³ Borazin, 5 g/m³ Pentaboran, 0,2 g/m³ des CBN-Heterofullerenfragmentderivates C₇₂H₄B₄N₄₈. Zum Unterschied wird das Pentaboran nicht zusammen mit der verdichteten Borazin-CBN-Heterofullerenfragmentderivat-Lösung zugegeben, sondern separat durch Argonbeladung mittels pentaborangefüllter Gaswaschflasche. Der entsprechend Beispiel 8 aufgearbeitete Extrakt der festen Hochtemperaturpyrolyseprodukte, eine rote feinteilige Masse, enthält zum überwiegenden Anteil das CBN-Heterofulleren C₇₂B₄₀N₄₈, einem Heterofulleren mit tetraedersymmetrischem Aufbau und nach Symmetrie und Atomzahl dem tetraedersymmetrischen Fulleren C₁₆₀ entsprechend. Das CBN-Heterofulleren C₇₂B₄₀N₄₈ ist ausschnitthaft in der Formel 22) dargestellt. Das CBN-Heterofulleren zeichnet sich dadurch aus, daß es je Molekül sechs B₆-Hexagonringe enthält.

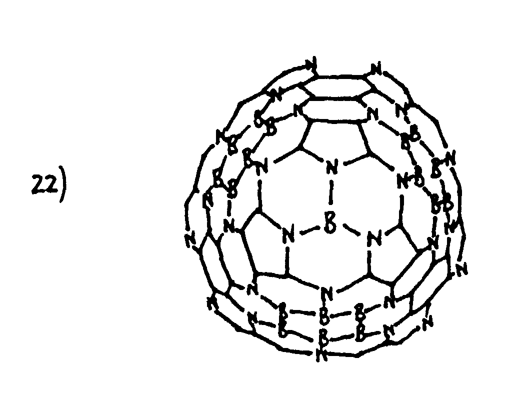

### Beispiel 12

100 g des octaedersymmetrischen Fullerenfragmentderivates C₁₉₂H₆₀N₄₈, dessen Molekülaufbau aus den Formelbildern 23) und 24) hervorgeht, die jeweils einen anderen Molekülausschnitt zeigen, das nach den Symmetrieeigenschaften dem octaedersymmetrischen Fulleren C₃₇₂ entspricht und das 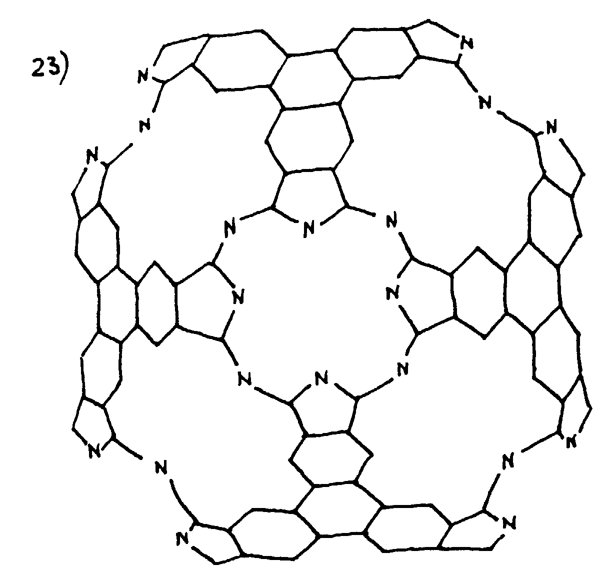 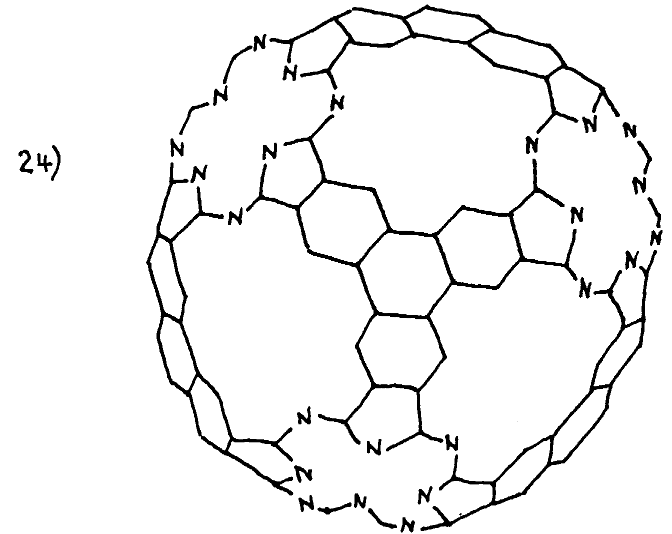 entsprechend der DE-A-4 114 536 hergestellt wurde, werden entsprechend Beispiel 3 zusammen mit Benzol pyrolysiert und die festen Pyrolyseprodukte entsprechend isoliert und aufgearbeitet. Als Produkt fällt ein bräunlicher Niederschlag an, der zu etwa 5 % aus dem oktaedersymmetrischen Fulleren C₃₇₂ besteht, das ausschnitthaft in der Formel 25) gezeigt wird und zwar mit Blickrichtung auf eine der acht"Oktaederflächen" des Fullerens. Das aus den Porphinfunktionen entstandene Hexagon-Pentagonnetzwerk, aus dem die "Ecken" des Oktaedersymmetrischen Fullerens entstanden sind, deren Position jeweils durch ein Pentagonpaar (in der Formel 25) schraffiert dargestellt) markiert ist, ist in der Formel 25) gepunktet dargestellt. Die Formel 25) zeigt beispielhaft nur ein Isomer aus der möglichen Isomerenschaar, die durch unterschiedliche Anordnung der Pentagonpaare zueinander gekennzeichnet ist, wobei in allen Isomeren die oktaedersymmetrische Molekültopographie bestehen bleibt.

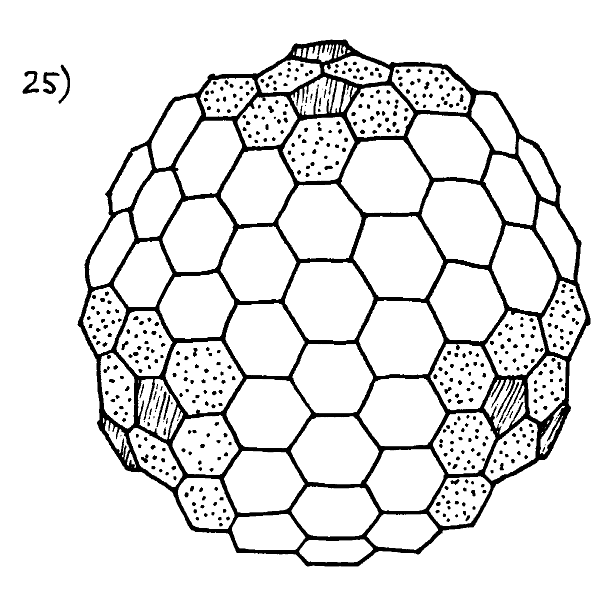

## Patentansprüche

1. Verfahren zur Herstellung von aus geschlossenem Hexagon- und Pentagonnetzwerk bestehenden Hohlmolekülen,
**dadurch gekennzeichnet, daß**
durch Pyrolyse aus Edukten Hohlmoleküle hergestellt werden,
a) die Kohlenstoff und Bor und/oder Stickstoff als Bestandteile des Hexagon- und Pentagonnetzwerkes enthalten, wobei Edukte verwendet werden, die diese Elemente enthalten oder
b) die Kohlenstoff und Bor und/oder Stickstoff als Bestandteile des Hexagon- und Pentagonnetzwerkes enthalten und bei denen Molekül symmetrie- und Molekülabmessungsparameter mit denen von mit Öffnungen im Hexagon- und Pentagonnetzwerk versehenen Hohlmolekülen von Eduktbestandteilen übereinstimmen, wobei Edukte verwendet werden, die Kohlenstoff und Bor und/oder Stickstoff und ein oder mehrere Fullerenfragmentderivate enthalten oder Edukte verwendet werden, die Kohlenstoff und Bor,und/oder Stickstoff und ein oder mehrere, Kohlenstoff und Bor und/oder Stickstoff enthaltende Heterofullerenfragmentderivate enthalten oder
c) die ausschließlich Kohlenstoff als Bestandteil des Hexagon- und Pentagonnetzwerkes enthalten und bei denen Molekülsymmetrie- und Molekülabmessungsparameter mit denen von mit Öffnungen im Hexagon- und Pentagonnetzwerk versehenen Hohlmolekülen von Eduktbestandteilen übereinstimmen, wobei Edukte verwendet werden, die Kohlenstoff und ein oder mehrere Fullerenfragmentderivate enthalten und
d) die Trennung des die Hohlmoleküle enthaltenden festen Reaktionsgemisches mittels üblicher Trenntechniken, insbesondere Extraktion mit organischen Lösungsmitteln und/oder durch Sorption, erfolgt.

2. Verfahren nach Anspruch 1, dadurch gekennzeichnet, daß die Pyrolyse der Edukte in der Gas- und/oder Aerosolphase vorgenommen wird.

3. Verfahren nach den Ansprüchen 1 und 2,
**dadurch gekennzeichnet, daß**
die Pyrolyse in der Gegenwart der Flammengase und/oder Flammenaerosole geschieht, die bei der unvollständigen Verbrennung entstehen.

4. Verfahren nach den Ansprüchen 1 bis 3,
**dadurch gekennzeichnet, daß**
die Pyrolyse bei einer Temperatur geschieht, bei der elementarer Kohlenstoff einen merklichen Dampfdruck hat, mindestens aber bei 2000°C.

5. Verfahren nach den Ansprüchen 1 bis 4,
**dadurch gekennzeichnet, daß**
als Oxidationsmittel für den unvollständigen Verbrennungsvorgang sauerstoff-, distickstoffmonoxid-, stickstoffmonoxid- oder stickstoffdioxidhaltige Gase oder deren Gemische eingesetzt werden.

6. Verfahren nach den Ansprüchen 1 bis 5,
**dadurch gekennzeichnet, daß**
als Edukte Kohlenwasserstoffe, Borazin, Hexamethylborazin, Bornitrid, Borcarbid, Pentaboran, 1-Pyrrolyl-boran, Stickoxide, Ammoniakgas, Harnstoff, BTX-Aromaten, Acetylen und aromatische Rußöle eingesetzt werden.

## Claims

1. Process for preparing hollow molecules consisting of a closed hexagonal and pentagonal network, characterised in that the starting materials are converted by pyrolysis into hollow molecules which
a) contain carbon, boron and/or nitrogen as constituents of the hexagonal and pentagonal network and/or
b) contain carbon, boron and/or nitrogen as constituents of the hexagonal and pentagonal network and in which the parameters of their molecular symmetry and molecular dimensions are identical to those of hollow molecules of starting material components provided with openings in the hexagonal and pentagonal network whereby starting materials are used which contain carbon and boron and/or nitrogen and one or more fulleren fragment derivates or starting materials are used which contain carbon and boron and/or nitrogen or one or more heterofulleren fragment derivates containing carbon and boron and/or nitrogen or which
c) exclusively contain carbon as constituent of the hexagonal and pentagonal network and in which the parameters of their molecular symmetry and molecular dimensions are identical to those of hollow molecules of starting material components provided with openings in the hexagonal and pentagonal network whereby starting materials are used which contain carbon and one or more fulleren fragment derivates and
d) the separation of the solid reactive mixture containing the hollow molecules is done by usual spearation technique eg. extraction with organic solvent and/or by sorption.

2. Process according to Claim 1, characterised in that the pyrolysis of the starting materials is carried out in the gas and/or aerosol phase.

3. Process according to Claims 1 and 2, characterised in that the pyrolysis takes place in the presence of flame gases and/or flame aerosols formed during incomplete combustion.

4. Process according to Claims 1 to 3, characterised in that the pyrolysis takes place at a temperature at which elemental carbon has a noticeable vapour pressure, but at at least 2000°C.

5. Process according to Claims 1 to 4, characterised in that the oxidising agents used for the incomplete combustion process are gases containing oxygen, dinitrogen monoxide, nitrogen monoxide or nitrogen dioxide or mixtures thereof.

6. Process according to Claims 1 to 5, characterised in that hollow molecules containing carbon, boron and nitrogen as constituents of the hexagonal and pentagonal network are prepared by using starting materials containing these elements.

## Revendications

1. Procédé de préparation de molécules creuses consistant en des réseaux hexagonaux et pentagonaux fermés, procédé caractérisé en ce que des molécules creuses sont produites à partir de composés de départ par pyrolyse.
a) qui contiennent du carbone et du bore et/ou de l'azote comme constituant(s) des réseaux hexagonaux et pentagonaux, en utilisant des composés de départ contenant ces éléments, ou
b) qui contiennent du carbone et du bore et/ou de l'azote comme constituant(s) des réseaux hexagonaux et pentagonaux et dans lesquels les paramètres de symétrie moléculaire et de dimension moléculaire coïncident avec ceux des molécules creuses formées de composantes des composés de départ et munies d'ouvertures dans le réseau hexagonal et pentagonal, en utilisant des composés de départ qui contiennent du carbone et du bore et/ou de l'azote et un ou plusieurs dérivés fragmentaires du fullérène ou en utilisant des composés de départ qui contiennent du carbone et du bore et/ou de l'azote et un ou plusieurs dérivés fragmentaires d'hétérofullérène contenant du carbone et du bore et/ou de l'azote, ou
c) qui contiennent exclusivement du carbone comme constituant du réseau hexagonal et pentagonal et dans lesquels les paramètres de symétrie moléculaire et de dimension moléculaire coïncident avec ceux de molécules creuses formées de composants des composés de départ et contenant des ouvertures dans le réseau hexagonal et pentagonal, de sorte que l'on utilise des composés de départ qui contiennent du carbone et un ou plusieurs dérivés fragmentaires du fullérène, et
d) la séparation du mélange réactionnel solide contenant les molécules creuses et réalisée à l'aide de techniques usuelles de séparation, notamment de l'extraction avec des solvants organiques et/ou par sorption.

2. Procédé selon la revendication 1, caractérisé en ce que l'on effectue la pyrolyse des composés de départ en phase gazeuse ou en phase d'aérosol.

3. Procédé selon les revendications 1 et 2, caractérisé en ce que la pyrolyse a lieu en présence de gaz de flamme et/ou d'aérosol de flamme apparaissant dans le cas d'une combustion incomplète.

4. Procédé selon les revendications 1 à 3, caractérisé en ce que la pyrolyse a lieu à une température à laquelle le carbone élémentaire possède une pression notable de vapeur, mais au moins à 2000 °C.

5. Procédé selon les revendications 1 à 4, caractérisé en ce que l'on utilise, comme oxydant pour le processus de combustion incomplète, des gaz contenant de l'oxygène, du monoxyde de diazote, du monoxyde ou du dioxyde d'azote, ou leurs mélanges.

6. Procédé selon les revendications 1 à 5, caractérisé en ce qu'on utilise, comme composés de départ, des hydrocarbures, de la borazine, de l'hexaméthylborazine, du nitrure de bore, du carbure de bore, du pentaborane, du 1-pyrrolyl-borane, des oxydes de l'azote, du gaz ammoniac, de l'urée, des hydrocarbures aromatiques de type BTX (benzène-toluène-xylènes), de l'acétylène et des huiles aromatiques fuligineuses (contenant de la suie ou provenant d'une suie).
